(19) **Europäisches Patentamt / European Patent Office / Office européen des brevets**

(11) **EP 2 426 615 A1**

(12) # EUROPEAN PATENT APPLICATION

(43) Date of publication:
**07.03.2012 Bulletin 2012/10**

(51) Int Cl.:
*G06F 19/24* *(2011.01)*

(21) Application number: **10008234.6**

(22) Date of filing: **06.08.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicants:
• **Roche Diagnostics GmbH**
  **68305 Mannheim (DE)**
  Designated Contracting States:
  **DE**
• **F. Hoffmann-La Roche AG**
  **4070 Basel (CH)**
  Designated Contracting States:
  **AL AT BE BG CH CY CZ DK EE ES FI FR GB GR HR HU IE IS IT LI LT LU LV MC MK MT NL NO PL PT RO SE SI SK SM TR**

(72) Inventor: **The designation of the inventor has not yet been filed**

(74) Representative: **Bösl, Raphael Konrad**
  **Isenbruck Bösl Hörschler LLP**
  **Patentanwälte**
  **Prinzregentenstrasse 68**
  **81675 München (DE)**

(54) **Evaluation device for assisting in the assessment of colorectal cancer**

(57)    The present invention relates to an evaluation tool (100) for assisting in the assessment of colorectal cancer. A method of operating an evaluation device (100) for evaluating measurement results for a multiplicity of biochemical markers (106) related to a presence or absence of colorectal cancer in an individual (112) comprises the steps of accepting measurement results indicative of a concentration of each of the markers Seprase, CEA, Ferritin, OPNT, and Anti-p53 (106); determining, based on the accepted measurement results, a score related to the presence or absence of colorectal cancer in the individual, wherein the determination includes calculating at least one interaction term comprising a multiplication of the measurement results for at least two of the markers; and outputting (108, 110) the determined score as a basis for use in assessing the presence or absence of colorectal cancer.

EP 2 426 615 A1

Fig. 1

**Description**

<u>Technical Field</u>

The present invention relates to an evaluation tool for assisting in the assessment of colorectal cancer. More specifically, the invention relates to a method and device for evaluating measurement results for a multiplicity of biochemical markers related to a presence or absence of colorectal cancer in an individual, and to a respective evaluation device.

Colorectal cancer (CRC) is among the most prevalent cancer types. As for other cancer types, CRC is often only detected when it becomes symptomatic. This is normally the case when patients already are in a rather late stage of disease progression. However, an early diagnosis of CRC translates into a much better prognosis. Colorectal cancer frequently progresses from adenomas (polyps) to malignant carcinomas. A best prognosis is for those patients diagnosed at the adenoma stage. For example, studies show that patients diagnosed in an early stage (if treated properly) have more than 90 percent (%) chance of survival 5 years after diagnosis as compared to a 5-years survival rate of only 10 % for patients diagnosed when distant metastases are already present.

Current detection methods, in particular imaging methods such as x-ray or nuclear resonance imaging are not normally useful as a general screening tool for mass-screening. This is because such methods are very costly and thus not affordable to health care systems for a general and broad use in mass-screenings of large numbers of subjects, particularly for subjects without any tumour symptoms. Moreover, even for the surveillance of patients already in therapy such methods are costly.

Cancer cells are characterized by the production of cancer-associated marker proteins. Cancer-associated proteins are found both in the tissues and in the bodily fluids of an individual carrying cancer cells. However, their levels usually are low at early stages of the carcinogenic progress and increase during the disease progression; in rare cases proteins are observed showing even a decreasing level in the course of disease progression. Thus, the sensitive detection of such proteins is another important approach for the diagnosis of cancer, for example and in particular for an early stage diagnosis of cancer.

The ideal scenario for a diagnosis would be a situation wherein a single event or process would cause the respective disease, as is the case, e.g., in infectious diseases. In all other cases correct diagnosis can be very difficult, especially when the aetiology of the disease is not fully understood. This is the case for many cancer types, including CRC. Practically, no biochemical marker is diagnostic with 100 % specificity and at the same time 100 % sensitivity for a given multifactorial disease, including CRC.

While there are single assays available which are used for the detection of CRC, their ability for diagnostic purposes is often not sufficient. More particularly, typically one or both of the sensitivity and specificity of single marker assays is often not sufficient, especially in the detection of early stages of CRC. In particular for a mass screening, a high specificity is required, as a poor specificity translates to a high number of unnecessary secondary investigations, like colonoscopy, which is not acceptable for a mass-screening. On the other hand, even for a high specificity, also the sensitivity has to be as high as possible in order that, for a mass screening, a reasonable number of individuals can be identified in the early stages of CRC, i.e. the sensitivity should be at least as high as 50 % for a specificity of 90 % or even 95 %.

A further approach is to identify several biochemical markers preferably detectable in body fluids such as blood or serum or plasma. It has turned out that such a combination of single markers may lead to an increased sensitivity. Such a marker panel for CRC has been described in the WO 2009/074276 A2. A measurement of the concentration and/or activity of a Seprase polypeptide and/or fragments thereof and of either Anti-p53 and/or Osteopontin (OPNT) and/or Ferritin is proposed and it is further proposed to use the combined measurement results for an assessment of CRC. However, it is to be noted that such multi-marker assay kits also lead to correspondingly increased costs which is relevant especially for mass screenings.

Even if for a combination of five markers a sensitivity of, e.g., 68 % can be reached at a specificity of 95 %, a further increase in sensitivity would be desirable. On the other hand, each marker to be considered still further for the assessment increases the costs further. For example, an increase in sensitivity from 68 % to 69 % at a specificity of 95 % may be reachable by including a sixth marker, however, this would increase the costs of the required analytical kits correspondingly, while at the same time the sensitivity increases only to a very small degree. Thus, the approach of using more and more markers for a more and more reliable detection of CRC is eventually limited by (cost-)efficiency reasons.

<u>Summary of the Invention</u>

It is one object of the present invention to provide a technique for CRC assessment, which on the one hand allows a detection of a presence or absence of CRC with a reasonable high specificity and/or sensitivity, and which on the other hand is practically feasible and efficient in terms of costs and complexity and is thus applicable for, e.g., an early detection of CRC by mass-screening of an asymptomatic population as well as for the surveillance of patients who undergo surgery.

The above object is achieved by a method of operating an evaluation device for evaluating measurement results for a multiplicity of biochemical markers related to a presence or absence of colorectal cancer in an individual. The method comprises the steps of accepting measurement results indicative of a concentration of each of the markers Seprase, CEA, Ferritin, OPNT, and Anti-p53; determining, based on the accepted measurement results, a score related to the presence or absence of colorectal cancer in the individual,; and outputting the determined score as a basis for use in assessing the presence or absence of colorectal cancer.

The determination includes calculating zero, one or more single terms, wherein a single term represents the measurement result for exactly one marker. The determination includes calculating one or more interaction terms, wherein an interaction term represents a multiplication of the measurement results for at least two of the markers. In the determination, each of the accepted measurement results for the markers is included in at least one of a single term and one or more interaction terms.

For example, the score may be used as a basis in an assessment related to the presence or absence of colorectal cancer in the individual.

The term "marker" or "biochemical marker" as used herein refers to a molecule to be used as a target for analyzing a patient's test sample. Examples of such molecular targets are proteins or polypeptides. Proteins or polypeptides used as a marker in the present invention are contemplated to include naturally occurring variants of said protein as well as fragments of said protein or said variant, in particular, immunologically detectable fragments. Immunologically detectable fragments preferably comprise at least 6, 7, 8, 10, 12, 15 or 20 contiguous amino acids of said marker polypeptide. One of skill in the art would recognize that proteins which are released by cells or present in the extracellular matrix may be damaged, e.g., during inflammation, and could become degraded or cleaved into such fragments. Certain markers are synthesized in an inactive form, which may be subsequently activated by proteolysis. As the skilled artisan will appreciate, proteins or fragments thereof may also be present as part of a complex. Such complex also may be used as a marker in the sense of the present invention. Variants of a marker polypeptide are encoded by the same gene, but may differ in their isoelectric point (=PI) or molecular weight (=MW), or both e.g., as a result of alternative mRNA, pre-mRNA processing or protein processing. The amino acid sequence of a variant is to 95% or more identical to the corresponding marker sequence. In addition, or in the alternative a marker polypeptide or a variant thereof may carry a post-translational modification. Preferred posttranslational modifications are glycosylation, acylation, and/or phosphorylation.

The term "Seprase" as used herein may comprise seprase polypeptide and/or fragments thereof, which may refers to monomeric or multimeric, particularly dimeric polypeptides. Further, seprase polypeptide and/or fragments thereof may particularly refer to soluble forms of seprase and/or fragments thereof, particularly antiplasmin-cleaving enzyme (APCE) as well as to seprase and/or seprase fragments present in form of a complex with another polypeptide.

Seprase polypeptides, particularly soluble forms of seprase polypeptides and/or fragments thereof, are preferably detected in appropriate samples, particularly in body fluids. Preferred samples are body fluids, such as blood, plasma, serum, sputum and urine. Preferably, the sample is derived from a human subject, e.g. a tumor patient or a person in risk of a tumor or a person suspected of having a tumor.

When measuring Seprase, it is understood that the concentration and/or activity of a seprase polypeptide and/or fragments thereof is determined in an analytical apparatus. For example, Seprase may be detected in a corresponding assay. Assays for the measurement of seprase activity are e.g. described by Lee et al., Blood 107 (2006) 1397-1404.

Piñeiro-Sánchez, J. Biol. Chem. 272 (1997) 7595-7601, found that an increased expression of Seprase correlates with the invasive phenotype of human melanoma and carcinoma cells. Henry et al., Clin. Cancer Res. 13 (2007) 1736-1741 describe that human colon tumor patients having high levels of stromal seprase are more likely to have aggressive disease progression and potential development of metastases or recurrence.

As indicated above the term seprase also relates to complex-bound seprase, e.g. to the DPPIV-seprase-complex. Human dipeptidyl peptidase IV (= DPPIV), which is also known as CD26, is a 110 kDa cell surface molecule. The amino acid sequence of human DPPIV protein comprises 766 amino acids (Swissprot database Accession No. P27487). It contains intrinsic dipeptidyl peptidase IV activity which selectively removes N-terminal dipeptide from peptides with proline or alanine in the third amino acid position. It interacts with various extracellular molecules and is also involved in intracellular signal transduction cascades. The multifunctional activities of human DPPIV are dependent on cell type and intracellular or extracellular conditions that influence its role as a proteolytic enzyme, cell surface receptor, co-stimulatory interacting protein and signal transduction mediator. Human DPPIV has a short cytoplasmatic domain from amino acid position 1 to 6, a transmembrane region from amino acid position 7 to 28, and an extracellular domain from amino acid position 29 to 766 with intrinsic dipeptidyl peptidase IV (DPPIV) activity.

The uman soluble dipeptidyl peptidase IV (= soluble DPPIV) amino acid sequence comprises the amino acid positions 29 to 766 from Swissprot database Accession number P27487. The dimer of soluble DPPIV is a 170 kDa glycoprotein consisting of two identical monomeric soluble DPPIV units.

Membrane bound human DPPIV/Seprase protein complex is formed of a 220 kDa DPPIV homodimer and a 170 kDa Seprase homodimer having an molecular weight of 410 kDa. Under certain conditions this complex may form a double complex having a molecular weight of 820 kDa. This membrane bound DPPIV/Seprase protein complexes have been reported by Ghersi, G. et al., J. Biol. Chem. 277 (2002) 29231-29241; Ghersi, G. et al., Adv. Exp. Med. Biol. 524 (2003) 87-94, and Ghersi, G. et al., Cancer Res. 66 (2006) 4652-4661 in human endothelial cells.

The term "soluble DPPIV/Seprase protein complex" (= DPPIV/Seprase) refers to the soluble complex formed of a soluble DPPIV homodimer (170 kDa) and a soluble Seprase homodimer (160 kDa) with a molecular weight of 330 kDa. Under certain conditions this complex may form a double complex having a molecular weight of 660 kDa.

CEA (carcinoembryonic antigen) is a monomeric glycoprotein (molecular weight approx. 180.000 Dalton) with a variable carbohydrate component of approx. 45-60% (Gold, P. and Freedman, S.O., J. Exp Med 121 (1965) 439-462). CEA, like AFP, belongs to the group of carcinofetal antigens that are produced during the embryonic and fetal period. The CEA gene family consists of about 17 active genes in two subgroups. The first group contains CEA and the Non-specific Cross-reacting Antigens (NCA); the second group contains the Pregnancy-Specific Glycoproteins (PSG).

CEA is mainly found in the fetal gastrointestinal tract and in fetal serum. It also occurs in slight quantities in intestinal, pancreatic, and hepatic tissue of healthy adults. The formation of CEA is repressed after birth, and accordingly serum CEA values are hardly measurable in healthy adults.

High CEA concentrations are frequently found in cases of colorectal adenocarcinoma (Fateh-Modhadam, A. et al. (eds.), Tumormarker und ihr sinnvoller Einsatz, Juergen Hartmann Verlag GmbH, Marloffstein-Rathsberg (1993), ISBN-3-926725-07-9). Slight to moderate CEA elevations (rarely> 10 ng/mL) occur in 20-50 % of benign diseases of the intestine, the pancreas, the liver, and the lungs (e.g. liver cirrhosis, chronic hepatitis, pancreatitis, ulcerative colitis, Crohn's Disease, emphysema (Fateh-Moghadam, A., et al., supra). Smokers also have elevated CEA values.

CEA concentrations within the normal range do not exclude the possible presence of a malignant disease.

Ferritin is a macromolecule with a molecular weight of at least 440 kD (depending on the iron content) and consists of a protein shell (apoferritin) of 24 subunits and an iron core containing an average of approx. 2500 $Fe^{3+}$ ions (in liver and spleen ferritin) (Wick, M. et al., Ferritin in Iron Metabolism - Diagnosis of Anemieas (second edition). Springer-Verlag 1995; ISBN 3-211-82525-8 and ISBN 0-387-82525-8) Ferritin tends to form oligomers, and when it is present in excess in the cells of the storage organs there is a tendency for condensation to semicrystalline hemosiderin to occur in the lysosomes. At least 20 isoferritins can be distinguished with the aid of isoelectric focusing (Arosio, P. et al., Heterogeneity of ferritin II: Immunological aspects. In: Albertini A, Arosio P, Chiancone E, Drysdale J (eds). Ferritins and isoferritins as biochemical markers. Elsevier, Amsterdam 1984;33-47). This microheterogeneity is due to differences in the contents of the acidic H and weakly basic L subunits. The basic isoferritins are responsible for the long-term iron storage function, and are found mainly in the liver, spleen, and bone marrow (Kaltwasser, J.P. et al., Serumferritin: Methodische und Klinische Aspekte. Springer Verlag (1980)). Acidic isoferritins are found mainly in the myocardium, placenta, and tumor tissue. They have a lower iron content and presumably function as intermediaries for the transfer of iron in various syntheses (Morikawa, K. et al., Leuk. Lymphoma 18(5-6) (1995) 429-433; Borch-Iohnson, B., Analyst 120(3) (1995) 891-903; Cook, J. et al., Adv. Exp. Med. Biol. 356 (1994) 219-228).

The determination of Ferritin is a suitable method for ascertaining the iron metabolism situation. Determination of Ferritin at the beginning of therapy provides a representative measure of the body's iron reserves. Clinically, a threshold value of 20 $\mu$g/L (ng/mL) has proved useful in the detection of prelatent iron deficiency. This value provides a reliable indication of exhaustion of the iron reserves that can be mobilized for hemoglobin synthesis. When the Ferritin level is elevated and the possibility of a distribution disorder can be ruled out, this is a manifestation of iron overloading in the body. 400 $\mu$g/L (ng/mL) ferritin is used as the threshold value. Elevated Ferritin values are also encountered with the following tumors: acute leukemia, Hodgkin's disease and carcinoma of the lung, liver and prostate. The determination of Ferritin has proved to be of value in liver metastasis. Studies indicate that 76% of all patients with liver metastasis have Ferritin values above 400 $\mu$g/L (ng/mL). Reasons for the elevated values could be cell necrosis, blocked erythropoiesis or increased synthesis in tumor tissue.

On the other hand it was described as characteristic, that serum Ferritin levels did not increase in gastrointestinal cancer (Niitsu, Y. et al., Rinsho Ketsueki 21 (1980) 1135-1143). Furthermore, mean serum Ferritin levels have been reported to be significantly lower in patients with advanced colorectal cancer than in controls (Kishida, T. et al., J. Gastroenterol. 29 (1994) 19-23). The decrease of serum Ferritin levels is caused by continous bleeding and this blood loss is related to the size and site of the tumor (Feng, L. et al., J. Gastroenterol. 34 (1999) 195-199).

Osteopontin (OPNT) OPNT is found in normal plasma, urine, milk and bile (US 6,414,219; US 5,695,761; Denhardt, D.T. and Guo, X., FASEB J. 7 (1993) 1475-1482; Oldberg, A., et al., PNAS 83 (1986) 8819-8823; Oldberg, A., et al., J. Biol. Chem. 263 (1988) 19433-19436; Giachelli, C.M., et al., Trends Cardiovasc. Med. 5 (1995) 88-95). The human OPNT protein and cDNA have been isolated and sequenced (Kiefer M.C., et al., Nucl. Acids Res. 17 (1989) 3306).

OPNT functions in cell adhesion, chemotaxis, macrophage-directed interleukin-10 (IL-10) suppression, stress-dependent angiogenesis, prevention of apoptosis, and anchorage-independent growth of tumor cells by regulating cell-matrix interactions and cellular signaling through binding with integrin and CD44 receptors. While constitutive expression of OPNT exists in several cell types, induced expression has been detected in T-lymphocytes, epidermal cells, bone cells, macrophages, and tumor cells in remodeling processes such as inflammation, ischemia-reperfusion, bone resorption, and tumor progression (reviewed by Wai, P.Y. and Kuo, P.C., J. Surg. Res. 121 (2004) 228-241).

OPNT is known to interact with a number of integrin receptors. Increased OPNT expression has been reported in a number of human cancers, and its cognate receptors (av-b3, av-b5, and av-b1 integrins and CD44) have been identified. In vitro studies by Irby, R.B., et al., Clin. Exp. Metastasis 21 (2004) 515-523 indicate that both endogenous OPNT expression (via stable transfection) as well as exogenous OPNT (added to culture medium) enhanced the motility and invasive capacity of human colon cancer cells in vitro. OPNT appeared to regulate motility though interaction with CD44. OPNT expression also reduced intercellular (homotypic) adhesion, which is regarded as a characteristic of metastatic cancer cells. Stable transfection of four poorly tumorigenic human colon cancer cell lines with OPNT also resulted in enhanced tumorigenicity in vivo with increased proliferation and increased CD31 positive micro vessel counts, concordant with the degree of OPNT expression.

"Anti-p53" refers to p53 antibodies. Such antibodies were discovered during the course of tumor-associated antigen screening in breast cancer patients without the knowledge of mutations of the p53 gene (Crawford, L. et al., Int. J. Cancer 30 (1982) 403-408). The tumor suppressor p53 is a phosphoprotein barely detectable in the nucleus of normal cells (Benchimol, S. et al., EMBO J. 1 (1982) 1055-1062). On cellular stress, particularly that induced by DNA damage, p53 can arrest cell cycle progression, thus allowing the DNA to be repaired or it can lead to apoptosis. In cancer cells that bear a mutant p53, this protein is no longer able to control cell proliferation, which results in inefficient DNA repair (Levine, A. Cell 88 (1997) 323-331). The most common changes of p53 in human cancer are point missense mutations, which are found in cancers of the colon, stomach, breast, lung, brain and esophagus (Greenblatt, M. et al., Cancer Res. 54 (1994) 4855-4878). It is estimated that p53 mutations is the most frequent genetic event in human cancers and accounts for more than 50% of cases. There is a very strong correlation between the frequency of p53 antibodies and the frequency of p53 mutations arguing that p53 mutations are involved in the appearance of these antibodies (Soussi, T. Cancer Res. 60 (2000) 1777-1788). P53 antibodies are found in human cancer patients with a specificity of 96%, but the sensitivity of such detection is only 30%.

Anti-p53 preferably is measured using two immuno-dominant peptides of the wild type p53 protein (Lubin et. al., Cancer Res. 53 (1993) 5872-5876).

The term "measurement" may comprise a quantitative measurement of a marker in a sample.

According to some realizations of the inventive method, a further marker or markers are measured. In one of these realizations, as a sixth tumour marker additionally CYFRA21-1 (cytoceratine-19) is measured and included in the score determination. This may lead to an even more reliable detection of CRC in terms of specificity and sensitivity, however at increased costs.

An assay for "CYFRA21-1" specifically measures a soluble fragment of cytokeratin 19 as present in the circulation. The measurement of CYFRA21-1 is typically based upon two monoclonal antibodies (Bodenmueller, H., et al., Int. J. Biol. Markers 9 (1994) 75-81). In the CYFRA21-1 assay from Roche Diagnostics, Germany, the two specific monoclonal antibodies (KS 19.1 and BM 19.21) are used and a soluble fragment of cytokeratin 19 having a molecular weight of approx. 30,000 Daltons is measured. Cytokeratins are structural proteins forming the subunits of epithelial intermediary filaments. Twenty different cytokeratin polypeptides have so far been identified. Due to their specific distribution patterns they are eminently suitable for use as differentiation markers in tumor pathology. Intact cytokeratin polypeptides are poorly soluble, but soluble fragments can be detected in serum (Bodenmueller, H., et al., supra).

CYFRA21-1 is a well-established marker for Non-Small-Cell Lung Carcinoma (NSCLC). The main indication for CYFRA21-1 is monitoring the course of non-small cell lung cancer (NSCLC) (Sturgeon, C., Clinical Chemistry 48 (2002) 1151-1159).

In primary diagnosis high CYFRA21-1 serum levels indicate an advanced tumor stage and a poor prognosis in patients with non-small-cell lung cancer (van der Gaast, A.., et al., Br. J. Cancer 69 (1994) 525-528). A normal or only slightly elevated value does not rule out the presence of a tumor. Successful therapy is documented by a rapid fall in the CY-FRA21-1 serum level into the normal range. A constant CYFRA21-1 value or a slight or only slow decrease in the CYFRA21-1 value indicates incomplete removal of a tumor or the presence of multiple tumors with corresponding therapeutic and prognostic consequences. Progression of the disease is often shown earlier by increasing CYFRA21-1 values than by clinical symptomatology and imaging procedures.

Slightly elevated values (up to 10 ng/mL) are rarely found in marked benign liver diseases and renal failure. There is no correlation with sex, age or smoking. The values for CY-FRA21-1 are also unaffected by pregnancy.

In one implementation of the method according to the invention, one of the at least one interaction terms may comprise a multiplication of the measurement results for Ferritin and Anti-p53.

According to one variant of the method, at least one of the at least one interaction terms comprises a multiplication of the measurement results for CEA and a further marker selected from the group of Seprase, Ferritin, OPNT and Anti-p53. For example, the score may be determined based on at least one of an interaction term comprising a multiplication (product) of the measurement results for CEA and Seprase, and an interaction term comprising a multiplication of the measurement results for CEA and OPNT.

A 'score' as understood herein is a natural or rational number as a result of a (mathematical) operation, by which the concentration (or expression) levels of two or more marker assays are combined, wherein the combination comprises at least one interaction term.

An interaction term is the product of two (or more) concentration levels of two (or more) markers. It may be necessary or advisable to build first the log of the concentration level for each marker before building the product.

The determination step may comprise, as a further operational step, performing an addition. For example, the determination step may comprise adding at least one single term with an interaction term. The measurement result for at least one marker may be used exclusively as an input for calculating one or more of the at least one interaction terms. In one variant, the measurement result for Anti-p53 is used exclusively as an input for calculating one of the interaction terms.

The measurement result for at least one marker may be used as an input for calculating one or more of the at least one interaction term, and may be used further as an input for at least one single term. For example, the measurement result for Seprase may be used as an input for calculating one interaction term and may be used as an input for one single term. Additionally or alternatively, the measurement result for CEA may be used as an input for calculating two interaction terms and may be used as an input for one single term. Additionally or alternatively, the measurement result for Ferritin may be used as an input for calculating one interaction term and may be used as an input for one single term. Additionally or alternatively, the measurement result for OPNT may be used as an input for calculating one interaction term and may be used as an input for one single term.

According to one realization of the inventive method, for determining the score, a weight is associated with one or more of the at least one interaction term and one or more single term. At least one weight may have a negative value. For example, at least one weight associated with a single term may have a negative value. In particular, a weight associated with the measurement result for Seprase in a single term may have a negative value. Additionally or alternatively, a weight associated with the measurement result of Ferritin in a single term may have a negative value. In one variant, at least one weight associated with an interaction term has a negative value. For example, a weight associated with an interaction term comprising a multiplication of the measurement results for CEA and Seprase may have a negative value.

According to one realization of the inventive method, the score may be determined based on an interaction term including the measurement results for CEA and OPNT, an interaction term including the measurement results for CEA and Seprase, and an interaction term comprising the measurement results for Ferritin and Anti-p53. Additionally or alternatively, the score may be determined based on at least one of a single term including the measurement result for CEA, a single term including the measurement result for Seprase, a single term including the measurement result for Ferritin, a single term including the measurement result for OPNT, and a single term including the measurement result for Anti-p53.

The step of outputting the determined score may comprise controlling accordingly any suitable output means including, for example, a screen, display device, printing device, storage means, e.g. computer-readable storage means, etc., and in fact any means suitable for providing the data immediately or for later use, for example, to medical personnel in order to allow an assessment related to the presence or absence of colorectal cancer in an individual based on the output data.

One implementation of the inventive method comprises the further steps of determining a significance of the determined score based on a predefined cut-off value; and outputting information indicative of the determined significance. In one variant of this implementation, at least one of sensitivity and specificity resulting from a particular predefined cut-off value are output together with the determined score and/or significance information.

A cut-off value for an individual marker or a marker combination can for example be determined from the testing of a group of healthy individuals. For example, a cut-off may be set to result in a specificity of 90%, 95%, or 98%. A value for an individual marker or a marker combination, respectively, above the cut-off value can for example be indicative for the presence of cancer. As the skilled artisan appreciates, for certain markers, like for Seprase and Ferritin, respectively, the same may be true for a value below a cut-off value.

According to one variant, the inventive method comprises the further steps of detecting a predetermined range of score values, into which the determined risks score falls, and outputting information indicative of the detected range. The outputting step may generally comprise outputting information related to the accepted measurement results and/or the cut-off value. The evaluation may be performed for multiple cut-off values. In this case, the multiple cut-off values may be output, optionally in conjunction with, e.g., corresponding significance information.

The above-mentioned objective is further achieved by a computer program product, which comprises programme code portions for performing the steps of one or more of the methods or method aspects described herein when the computer program product is executed on one or more computing devices, for example an evaluation device. The computer program product may be stored on a computer readable recording medium, such as a permanent or re-writable memory within or associated with a computing device or a removable CD-ROM, DVD or USB-stick. Additionally or alternatively, the computer program product may be provided for download to a computing device, for example via a data network such as the Internet or a communication line such as telephone link or wireless link. According to one aspect of the invention, a computer readable recording medium may have computer executable instructions for performing any of the methods and method aspects described herein.

The above-mentioned object is further achieved by an evaluation device adapted for evaluating measurement results for a multiplicity of biochemical markers related to a presence or absence of colorectal cancer in an individual. The evaluation device comprises a component adapted to accept measurement results indicative of concentration of each of the markers Seprase, CEA, Ferritin, OPNT, and Anti-p53; a component adapted to determine, based on the accepted measurement results, a score related to the presence or absence of colorectal cancer in the individual, wherein the determination includes calculating zero, one or more single terms, a single term representing the measurement result for exactly one marker, and calculating one or more interaction terms, an interaction term representing a multiplication of the measurement results for at least two of the markers;

wherein each of the accepted measurement results for the markers is included in at least one of a single term and one or more interaction terms; and a component adapted to output the determined score as a basis for use in assessing the presence or absence of colorectal cancer.

The evaluation device may be remotely connectable with an analytical apparatus adapted to perform measurements of multiplicity of biochemical markers.

The above-indicated objective in one embodiment is achieved by an analytical apparatus comprising assays for Seprase, CEA, Ferritin, OPNT, and Anti-P53, and further comprising an evaluation device as outlined above.

Advantages of the Invention

With regard to efforts for a more (cost-)efficient detection of CRC with at the same time increased reliability, the invention proposes not to focus on adding further and further markers to a canonical set of markers (although such set is proposed including Seprase, CEA, Ferritin, OPNT, and Anti-p53), but to instead put more weight on evaluating the results of measuring the limited set of five markers. As compared to simply summing up the measurement results of the markers ('single terms' in the notation as used herein), additional relevant information can be obtained by considering interaction terms comprising products of two (or, in principle, multiple) markers with each other.

The combination of single terms and interaction terms can be complex, for example the role of a single term might be to correct for the importance of an interaction term where both terms may comprise a particular marker. This can be expressed by a negative weight assigned to, e.g., the single term, while the interaction term has a positive weight assigned thereto.

The score provides for an integrated single value summarizing the complex relation of the multiple markers, thereby providing a basis to be used, probably besides other data, in an assessment of the presence or absence of CRC in an individual. According to the invention, a tool is provided (which can be a software tool or a hardware tool / device, or a combination of both) for providing the score and optionally further, accompanying data based on the accepted measurement results for the markers Seprase, CEA, Ferritin, OPNT, and Anti-p53.

In this way, an assessment of CRC can be performed which is efficient in terms of cost as it relies on measuring a well defined limited set of markers and which is reliable as the information available from the measurements is used in a complex way to arrive at a high specificity as well as a high sensitivity. In particular, it is more cost-efficient to provide an evaluation tool according to the invention than extending the set of markers from five markers to include another sixth (seventh, ...) marker in order to arrive at a high sensitivity. Thus, the invention is particularly useful for being applied for mass-screening of an asymptomatic population (but also for, e.g., a surveillance of patients already in therapy).

Short Description of the Figures

Further aspects and advantages of the invention will become apparent from the following description of particular embodiments illustrated in the figures, in which:

Fig. 1     schematically illustrates an exemplary analytical scenario including a first embodiment of an evaluation device according to the invention;

Fig. 2     is a block diagram illustrating functional components of the evaluation device of Fig. 1;

Fig. 3     is a flow diagram illustrating an operation of the evaluation device of Fig. 1;

Fig. 4     schematically illustrates an exemplary output of the evaluation device of Fig. 1; and

Fig. 5    schematically illustrates an analytical apparatus equipped with a second embodiment of an evaluation device according to the invention.

<u>Detailed Description of Preferred Embodiments</u>

Those skilled in the art will appreciate that functions explained hereinbelow may be implemented using individual hardware circuitry, using software functioning in conjunction with a programmed microprocessor or a general purpose computer, using an application specific integrated circuit (ASIC) and/or using one or more digital signal processors (DSPs). It will also be appreciated that when the current invention is described as a method, it may also be embodied in a computer processor and a memory coupled to the processor, wherein the memory is encoded with one or more programs that perform the methods disclosed herein when executed by the processor.

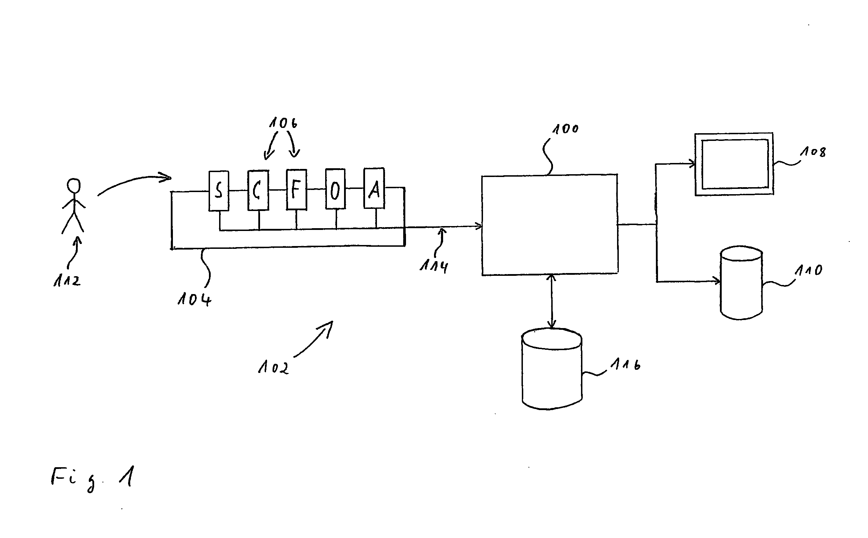

A particular embodiment of the invention will be described below with reference to the exemplary scenario outlined in Fig. 1. An evaluation device 100 is integrated into an analytical apparatus 102 further comprising reagent sets for measuring a multiplicity of analytes 104 including a multiplicity of assays 106, a display screen 108 and a storage device 110. Generally, the analytical apparatus 102 operates to analyse blood or serum of an individual 112, which may be a patient or another, asymptomatic, individual, e.g. one of many individuals tested during a mass-screening. As a specific example, the apparatus 102 may comprise a Roche Elecsys® 2010 System from Roche Diagnostics GmbH, Mannheim, Germany.

Screening is understood herein as the systematic application of a test to identify individuals e.g. at risk individuals, for indicators of a disease, e.g., the presence of colorectal cancer. Preferably the screening population is composed of individuals known to be at higher than average risk of cancer. For example, a screening population for colorectal cancer may be composed of individuals known to be at higher than average risk of colorectal cancer, e.g. individuals of an age at and above of 50, or at or above an age of 60.

The reagent sets 104 comprise in particular immunoassay reagents for measuring a concentration of Seprase, CEA, Ferritin, OPNT and Anti-p53 (depicted as S, C, F, O, A, respectively, 106, in Fig. 1). The analytical apparatus 102 is connected via an internal bus 114 to the evaluation device 100, such that the evaluation device 100 may receive information (in the form of an electrical signal) indicative of a measurement result from each of the assays 106.

During operation, which will be described in more detail below with reference to the following figures, the evaluation device 100 evaluates a score related to the presence or absence of colorectal cancer for the individual 112. When performing the evaluation, the evaluation device 100 may access predefined data in storage 116 associated with or forming an integral part of the analytical apparatus 102. Specific examples for the predefined data may comprise assessment-specific and/or region-specific data such as a specific cut-off value (the role of such data also being described in more detail below).

An evaluation result for the individual 112 may be output on one or both of the devices 108 and 110. An output to the screen 108 may include a score and optionally further related data in order to allow, e.g., medical personnel to assess a presence or absence of colorectal cancer in the individual 112. Alternatively or in parallel data related to the evaluation result may also be stored in storage 110, which may comprise a recording medium such as a DVD, for later diagnostic or prognostic use, monitoring of therapy, general documentation purposes, and/or further, e.g., statistical, evaluations to be performed using the analytical apparatus 102 or a different computing device.

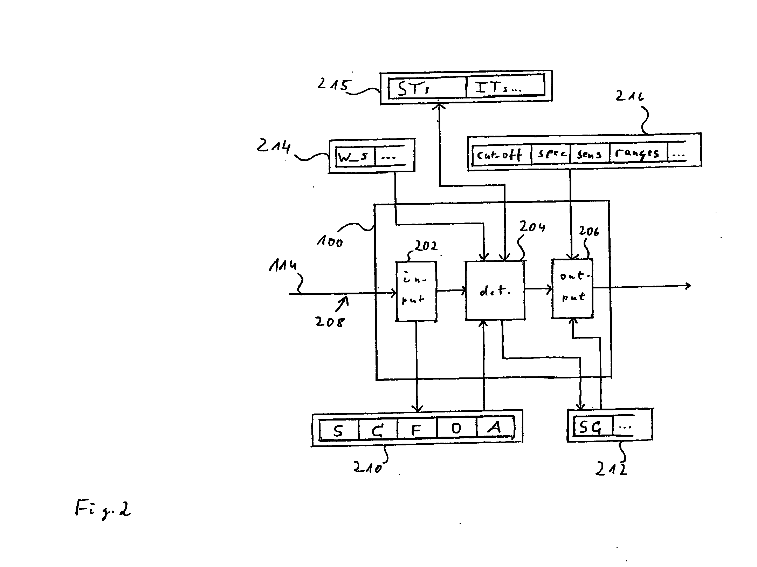

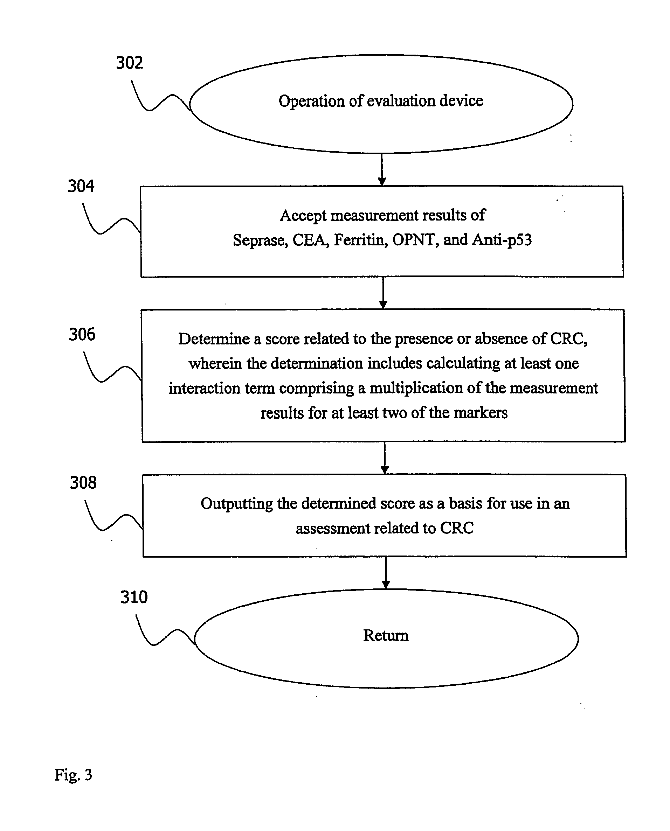

Fig. 2 is a block diagram schematically illustrating functional components of the evaluation device 100 of Fig. 1. The evaluation device 100 comprises an input component 202, a determination component 204, and an output component 206. An operation of the evaluation device 100 is described with reference to the flow diagram of Fig. 3. Generally, the evaluation device 100 operates 302 to evaluate the measurement results for the multiplicity of biochemical markers measured by the kit 104 and to provide, as an evaluation result, at least a score as a basis for use in an assessment related to the presence or absence of colorectal cancer in the individual 112.

In step 304, the input component 202 accepts measurement results 208 indicative of the concentration of the markers Seprase, CEA, Ferritin, OPNT, and Anti-p53. In the exemplary embodiment described here, the input component 202 in particular receives the measurement results via the internal data bus 114 from the analytical kit 104. The input component 202 stores the received measurement results in an associated storage area in a data record 210 in a data format as appropriate for further internal processing.

In step 306, the determination component 204 operates to determine a score based in the measurement results. The calculations performed by the determination component 204 will be described in more detail further below. The determination results are stored in a further data record 212. In step 308, the output component 206 is triggered by a trigger signal received from the determination component 204 and/or an operator of the analytical apparatus 100 to output the determined score and optionally further data to one or both of the devices 108 and 110 (cf. Fig. 1). In step 310, the operation stops, i.e. the evaluation device 100 is ready for further processing of, e.g. a next set of measurement results.

Referring now to the details of the calculations performed by the determination component 204, the following Equation 1 shows an example of such a calculation:

$$\text{(Eq. 1)} \quad SC = \; ST(CEA) + ST(Seprase) + ST(Ferritin) + ST(OPNT) +$$
$$IT(CEA, OPNT) + IT(CEA, Seprase) + IT(Ferritin, Anti\_p53) + IC$$

wherein SC denotes the calculated score, ST denotes a Single Term, IT denotes an Interaction Term, and IC denotes an Intercept. Each of the Single Terms referred to in Equation 1 represent the measurement result for one marker. The Single Terms are structurally defined in equation 2:

$$\text{(Eq. 2)} \quad ST(CEA) = W\_CEA * \log(CEA) ,$$
$$ST(Seprase) = W\_Seprase * \log(Seprase) ,$$
$$ST(Ferritin) = W\_Ferritin * \log(Ferritin) ,$$
$$ST(OPNT) = W\_OPNT * \log(OPNT) ,$$

wherein W denotes a particular weight assigned to the respective term, and log() is the logarithm of the measurement result for a particular marker as stored in the data record 210. The following Equation 3 specifies the structure of the Interaction Terms representing a multiplication of the measurement results for two markers in more detail:

$$\text{(Eq. 3)} \quad IT(CEA, OPNT) = W\_CO * \log(CEA) * \log(OPNT) ,$$

$$IT(CEA, Seprase) = W\_CS * \log(CEA) * \log(Seprase) ,$$
$$IT(Ferritin, Anti\_p53) = W\_FA * \log(Ferritin) * \log(Anti\_p53) ,$$

wherein W denotes a particular weight assigned to the respective term, and log() is the logarithm of the measurement result for a particular marker as stored in the data record 210.

As a particular example, the following Equation 4 identifies a set of weights which may be stored as constants in a corresponding data record 214 associated with the determination component 204:

$$\text{(Eq. 4)} \quad W\_CEA = -2.71 ,$$
$$W\_Seprase = -3.61 ,$$
$$W\_Ferritin = -1.76 ,$$
$$W\_OPNT = 1.14 ,$$
$$W\_CO = 0.62 ,$$
$$W\_CS = -0.023 ,$$
$$W\_FA = 0.232 ,$$
$$IC = 8.643 .$$

These weights are related to a representation of the measurement results particular units, namely Seprase measured in [nanogramm/millilitres], CEA measured in [nanogramm/millilitres], Ferritin measured in [nanogramm/millilitres], OPNT measured in [nanogramm/millilitres] and Anti-p53 measured in [nanogramm/millilitres].

As to the date of finishing of this document, the specific values of the individual weights as exemplarily illustrated in equation 4 have not been entirely settled. In particular, in order for an optimum sensitivity at a specificity of preferably 95 %, the presently preferred ranges for the weights are specified as follows in Equation 5:

$$(\text{Eq. 5}) \qquad W\_CEA = [-9, 3.1],$$
$$W\_Seprase = [-3.9, -1.6],$$
$$W\_Ferritin = [-2, -1.1],$$
$$W\_OPNT = [0.3, 2.3],$$
$$W\_CO = [-0.2, 1.3],$$
$$W\_CS = [-0.7, 0.6],$$
$$W\_FA = [0.1, 0.4],$$
$$IC = [-3.2, 13].$$

Within these ranges, combinations of weights can be found, which achieve a sensitivity above 60 %, and even above 70 % at a specificity of 95 %. One or more of such particular optimized sets of weights may be stored in the storage area 214 for use in the calculcations. These sets may be updated either manually or by a remote updating procedure.

Equation 1 sums - besides the Single Terms ST - additionally a number of Interaction Terms IT. It is to be noted that from the five markers under consideration, only four of these are represented in single terms, while Anti-P53 is only considered in an interaction term. However, each of the markers is considered at least once in an interaction term, which emphasizes the important role of considering such interactions for achieving reliable detection of CRC. CEA is included in two of the interaction terms, namely in conjunction with OPNT and in conjunction with Seprase.

It is further to be noted that, as indicated in Equation 4, various of the weights may have a negative value. For example, a high measurement result for Ferritin tends to increase the score as this marker is considered with a positive weight in an interaction term in conjunction with Anti-p53. On the other hand, a high measurement of Ferritin also lowers the score SC due to the negative weight of the single term for Ferritin. In this way, a negative weight in a single term may be seen as correcting the influence of a corresponding interaction term.

Based on the above Equations 1 - 4, the determination component 204 calculates the score SC. As intermediate results, the single terms STs and interaction terms ITs are calculated and temporarily stored in a buffer 215. The score is calculated as a value between 0 and 1 (in other embodiments, the calculations may be adapted to provide a score between other ranges, e.g. between 0 and 100, or between -1 and 1). Generally, a higher score indicates a higher risk of colorectal cancer being in fact present in the measured individual. Therefore one may perceive the score SC as a 'risk score'.

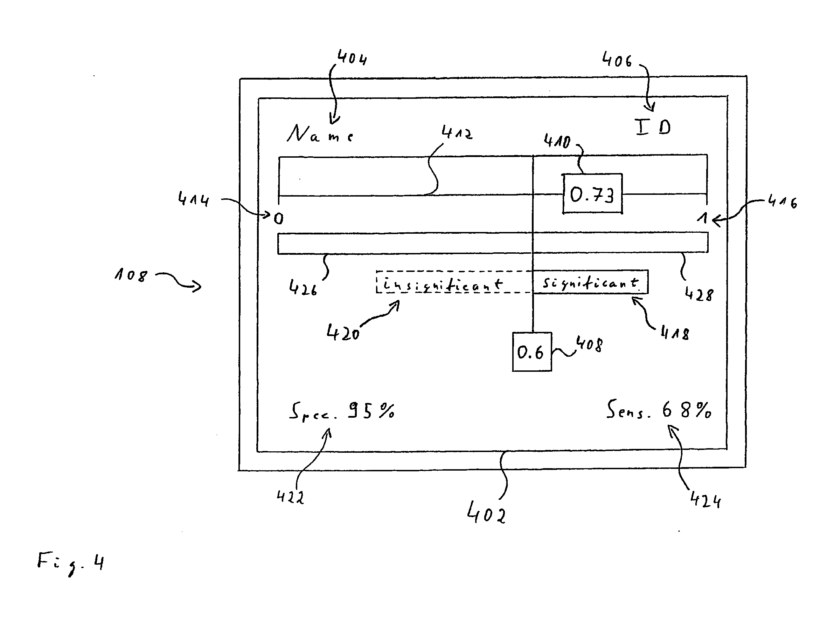

After having calculated the score, the determination component 204 stores the result in the data record 212. Further calculations related to the reliability of the determined score may be performed by one or both of the determination component 204 and output component 206. Here it will be assumed for simplicity that only the output component 206 performs these further calculations. This will be described with reference to Fig. 4 which illustrates an exemplary output 402 as it may be provided by the output component 206 to the screen 108 for display.

On top of the display 402, a name 404 and an ID-number 406 which may be related to the individual 112 (cf. Fig. 1) are displayed. Besides the calculated score, further data are presented on the display 402. A predefined cut-off value is stored in a data record 216 (cf. Fig. 2) in association with the evaluation device 100. The output component 206 compares the score of data record 212 whether it has a value above or below the cut-off value stored in record 216. Depending on the result, the display 402 is prepared for presentation on the screen 108. The predetermined cut-off value 408 is selected to be at a value of 0.6 in this example, which may follow from, e.g., a national regulation effective at the place of the individual 112. The cut-off value 408 is indicated on the display 402 in a box and as a line.

The cut-off value stored as stored in the data record 212 may comprise a single (combined) cut-off value for evaluating the significance of the determined score, but may additionally or alternatively comprise cut-off values for one or more of the measured markers. The results of separately comparing each of the measurement results with a corresponding marker-specific cut-off can also be combined to arrive at a significance result for the determined score. The data record 212 may also comprise different cut-off values related to different use cases; for example, different cut-offs may be provided as required for different regulatory regimes (e.g., performing the evaluation according to different national regulations). The cut-off value or values may be stored in the data record 212 by a manufacturer of the evaluation device 100, and/or may be administered, e.g. updated, by the manufacturer or an operator or user of the evaluation device 100 either manually or be installing an update package on the evaluation device or by a remote installation / update procedure.

The score 410 for the individual 112 may have been turned out to be (in this example) 0.73. The score 410 is indicated as a number in a box, the box being arranged in the display 402 in a geometrical relation to the cut-off value 408 such that the significance of the score 410 can be easily gathered. The presentation position of the score 410 is arranged on a line 412 reaching from a value of 0 (414) to a value of 1 (416) as the minimum and maximum possible score values. Thus, the arrangement of the score 410 to the right or to the left of the cut-off value 408 already indicates to a medical personnel or other user of the display 402 whether the result achieved from the measurements are significant. The example display 402 of Fig. 4 indicates that there is a significant risk of CRC being present in the individual 112, as the score 410 value of 0.73 is above of the cut-off value of 0.6 separating significant results from insignificant results (by the very definition of the cut-off value).

As a further aid in guiding the user of the display 402, significance information 418 is provided on the screen 108. Similar insignificance information 420 (sketched in a dashed line in Fig. 4 for reasons of illustration) may alternatively be presented in order to indicate an insignificant result, i.e. in case the score turns out to be below the predefined cut-off value. Still further, a specificity 422 and a sensitivity 424, the precise values of which are related to the predefined cut-off value 408, are presented on the screen in order to further assist a user of the display 402 in assessing the presence or absence of CRC in the individual 112.

Moreover the output component 206 operates to detect one of multiple predetermined ranges (also stored in association with the cut-off value in data record 216) into which the determined score falls. Colour bars 426 and 428 may be presented on the display 402 wherein, for example, the colour bar 426 may denote a range of scores below the cut-off value in that, e.g. the colour bar 426 may be green. The colour bar 428 may denote a range of scores above the cut-off value, and may in fact show a single colour such as red or yellow, or may show a colour changing from yellow near to the cut-off value 408 to red near to a score value of 1 (416). Alternatively, several ranges may be provided, e.g. a "yellow" range may reach from the cut-off value of (in this example) 0.6 to 0.75, while a further "red" range of highly significant score values ranges from 0.75 towards 1. The box showing the determined score 410 may have a colour corresponding to its position along the colour bar 428. Thus, the score value of 0.73 in the example of Fig. 4 would be indicated in yellow. However, a score value of 0.77, for example, would be indicated in red signalling a high risk for the presence of CRC in the individual 112 with a high significance.

It is to be noted that the presentation of data on the display 402 is not intended in any respect to replace a diagnosis or a decision on further steps to be taken (e.g. whether or not a further assessment has to be taken), which can only be performed by an experienced medical person. The evaluation device 100 is merely intended to assist by providing its corresponding output as a basis for use in an assessment related to the presence or absence of colorectal cancer in an individual. The term "as a basis for use in an assessment of colorectal cancer" is to be understood such that the invention may aid a physician, e.g., when establishing or confirming the absence or presence of CRC, and/or may aid the physician in the prognosis, the detection of recurrence (follow-up of patients after surgery) and/or the monitoring of treatment, especially of chemotherapy. It will be appreciated that an "assessment" means to decide the presence or absence of colorectal cancer with a certain likelihood or predictive value.

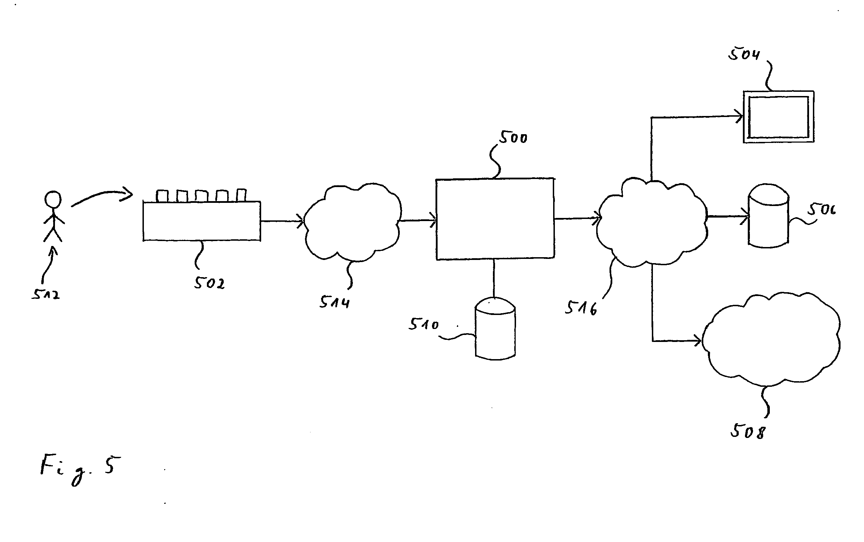

Fig. 5 illustrates another exemplary scenario in which a second embodiment 500 of an evaluation device according to the invention is operable. The evaluation device 500 is connected with an analytical apparatus 502, with output devices 504, 506 and may be connected with other processing or computing equipment via a network 508. The evaluation device 500 further is connected with a database 510. Generally, the analytical apparatus 502 measures blood or serum of an individual 512, which may be an individual such as a patient or an asymptomatic person. The analytical apparatus 502 may comprise a multiplicity of immunoassays for a detection of tumour markers, and in particular may comprise assays for Seprase, CEA, Ferritin, OPNT, and Anti-p53. As a specific example, the apparatus 502 may comprise a Roche Elecsys® 2010 Systems from Roche Diagnostics GmbH, Mannheim, Germany.

Measurement results for the above-indicated markers may be transmitted from the analytical apparatus 502 to the evaluation device 500 via a network 514 which may comprise at least one Local Area Network (LAN) and Wide Area Network (WAN). The network 514 may also comprise one or more wireless connections. Consequently, the evaluation device 500 may be located near to the analytical apparatus 502, but may also be located remote. In fact, the analytical apparatus 502 and evaluation device 500 may be located in one or different rooms of a building or in different buildings, or may even be located in different geographical regions.

An operation of the evaluation device 500 may be similar to what has been described above with reference to the device 100 illustrated in Figs. 1 to 4, and these aspects will not be repeated here. Generally, the evaluation device 500 may operate to evaluate a score related to the presence or absence of colorectal cancer in the individual 512. While not being explicitly shown in Fig. 5, the evaluation device 500 may be adapted to process such evaluations parallel or quasi-parallel for many individuals. For example, the evaluation device may be connected with many apparatuses such as apparatus 502. In this way, the single evaluation device 502 (which may be implemented on a server, for example) may serve many different locations equipped (only) with analytical apparatuses. Vice versa, such a configuration is efficient as there is no need to provide one evaluation device for one analytical apparatus, provided the analytical apparatuses can be connected to a network such as network 514 in Fig. 5.

When performing evaluations, the evaluation device 500 may access predefined data in database 510. As one specific example, the analytical apparatus 502 may be located in a particular area of law; then the evaluation device 500 may access corresponding data as stored in the database 510, and may for example query a specific cut-off value prescribed according to a national regulation.

Evaluation result data including at least the determined score for the individual 112 may be output by the evaluation device 500 via a network 516 to the screen or display device 504 for presentation and immediate use by a user such as a medical person in an assessment related to the presence or absence of CRC of the individual 112. Additionally or alternatively the evaluation result data may be output to storage media 106, for example for later use, for documentation purposes, or for further, e.g., statistical evaluations. The evaluation device 500 may be adapted to provide many scores or, more general, many evaluation results in parallel to either one or both of the screen 504 and storage 506.

The evaluation device 500 may also share part or all of the evaluation result data with other computing devices via network 508, which may comprise a LAN and/or WAN, and may, e.g., be similar or identical to network 514 or 516 (e.g., the Internet). As one example, such computing device may be a remote personal computer used by a medical person, e.g. a medical practitioner consulted by the individual 512 after its blood has been analyzed; this configuration would also allow a remote diagnosis, for example. It is to be understood that, in one example configuration, one or both of display 504 and storage 506 are located near to the analytical apparatus 502 and/or individual 512, while the evaluation device 500 is remotely located. Correspondingly, the network 516 may comprise a LAN and/or WAN.

It is to be understood that an evaluation device according to the invention such as, for example, illustrated by the embodiment 500 may be implemented on a computing device such as, for example, a general purpose personal computer or workstation, a general purpose notebook computer, a pocket computer, handheld computer, or a PDA (Personal Digital Assistant). Also, an evaluation device according to the invention such as, for example, illustrated by the embodiment 100 may be implemented on dedicated computing devices intended specifically for use in a medical environment such as a computing device adapted for forming part of an analytical system such as, for example, a Roche Elecsys® system. An evaluation device according to the invention may be seen as a tool which is implemented in software and/or hardware as appropriate for a particular application, and which is provided as a tool for aiding in the assessment of CRC by implementing the relevant evaluation / determination procedures or routines which are required in order to achieve a desired specificity and sensitivity with a limited set of markers.

For further illustration of the invention, examples of a Training Study and a Validation Study are described below. A training dataset and a validation dataset were taken, consisting of serum samples collected prospectively in two European multi-center studies. The first study recruited patients at gastroenterology units in an average-risk screening population, which underwent a preventive check by colonoscopy. From each participant a colonoscopy was performed at the participating centers with preparation and sedation used at each site. The serum samples were collected shortly before the colonoscopy was performed. The size and location of each lesion were recorded. Pathologist examined each surgical resection specimen on site to determine the diagnosis and the respective staging. Due to the low incidence of approx. 0.05% of CRC patients within the preventive screening population cancer patients were additionally recruited in a second prospective study at different surgery units. In this study the serum samples were also collected before surgery or colonoscopy. The diagnosis of colorectal cancer was confirmed by pathological staging of each patient by pathologists on site. The research protocols for both studies were reviewed and approved by the appropriate ethics committees and all participants gave written informed consent.

The clinical samples from both multi-center studies were compiled for the evaluation of the markers mentioned before (cf. Figure 1) resulting for the training data set in a control cohort "A" and a CRC cohort "B", respectively. In detail:

> **A:** Control cohort with 266 patients. (No patients with adenoma or inflammatory bowel diseases had been included into the control cohort.)
> **B:** CRC cohort with 301 colorectal cancer patients.

For the validation dataset cohort A consisted of controls with 1102 patients and cohort B consisted of 192 colorectal cancer patients. For each patient, logarithmized measurement values of Seprase, CEA, Ferritin, OPNT and anti-p53 were obtained. From these data the multivariate method was deduced on the training dataset in a Monte-Carlo-Cross-Validation design, resulting in the weights given below in Table 1 for the single terms and the interaction terms.

Table 1: Weights of the single terms and interaction terms derived in the training study.

| Term | Marker | Weight |
|------|--------|--------|
| Intercept | Intercept | 8.64 |
| Single Term | Log.CEA | -2.71 |
| Single Term | Log.FERR | -1.76 |
| Single Term | Log.OPN.T | 1.14 |
| Single Term | Log.Seprase | -3.61 |
| Interaction Term | Log.CEA*log.OPN.T | 0.62 |
| Interaction Term | Log.CEA*log.Seprase | -0.02 |
| Interaction Term | Log.FERR*log.anti.p53 | 0.23 |

Given the weights of Table 1, a cutoff of 0.84 on the scores was derived, in order to achieve a specificity of 95%. With this cutoff the sensitivity on the training study was 74%.

To the data of the validation study the method with the weights given in Table 1 and the respective cutoff on the scores was applied, too, resulting in a specificity of 95% and a sensitivity of 61 %.

Regarding two individual patients, we obtain the following measurement results and the calculated scores:

| Patient Cohort | CEA | FERR | Seprase | OPN.T | Anti.p53 | Calculated Score | Risk Group |
|----------------|-----|------|---------|-------|----------|------------------|------------|
| Control | 4.48 | 106.8 | 44.3 | 285.6 | 40 | -1.79 | Low Risk |
| CRC | 6.03 | 5.7 | 25.5 | 448.9 | 40 | 4.10 | High Risk |

While the current invention has been described in relation to some of its currently preferred embodiments, it is to be understood that this description is intended for illustrative purposes only. Accordingly it is intended that the invention shall be limited only by the scope of the claims appended hereto.

**Claims**

1. A method of operating an evaluation device (100, 500) for evaluating measurement results for a multiplicity of biochemical markers related to a presence or absence of colorectal cancer in an individual (112, 512), comprising the steps of

- accepting (304) measurement results indicative of a concentration of each of the following markers:

    o Seprase,
    o CEA,
    o Ferritin,
    o OPNT, and
    o Anti-p53;

- determining (306), based on the accepted measurement results, a score (410) related to the presence or absence of colorectal cancer in the individual (112, 512), wherein the determination includes

    o calculating zero, one or more single terms, a single term representing the measurement result for exactly one marker, and
    o calculating one or more interaction terms, an interaction term representing a multiplication of the measurement results for at least two of the markers;
    o wherein each of the accepted measurement results for the markers is included in at least one of a single term and one or more interaction terms; and

- outputting (308) the determined score (410) as a basis for use in assessing the presence or absence of colorectal cancer.

2. The method of claim 1,
wherein one of the at least one interaction terms comprises a multiplication of the measurement results for Ferritin and Anti-p53.

3. The method of claim 1 or 2,
wherein the score is determined based on at least one of an interaction term comprising a multiplication of the measurement results for CEA and Seprase, and an interaction term comprising a multiplication of the measurement results for CEA and OPNT.

4. The method of any of the preceding claims,
wherein the measurement result for at least one marker is used exclusively as an input for calculating one or more of the at least one interaction terms.

5. The method of claim 4,
wherein the measurement result for anti-p53 is used exclusively as an input for calculating one of the interaction terms.

6. The method of any of the preceding claims,
wherein the measurement result for at least one marker is used as an input for calculating one or more of the at least one interaction term, and is used as an input for at least one single term.

7. The method of any of the preceding claims,
wherein for determining the score a weight is associated with one or more of the at least one interaction term and one or more single term.

8. The method of claim 7,
wherein at least one weight has a negative value.

9. The method of any of the preceding claims,
comprising the further steps of

- determining a significance (418) of the determined score (410) based on a predefined cut-off value (408); and
- outputting information (418) indicative of the determined significance.

10. The method of any of the preceding claims,
comprising the further steps of

- detecting a predetermined range (426, 428) of score values, into which the determined score (410) falls, and
- outputting information (428) indicative of the detected range.

11. The method of any of the preceding claims,
comprising the further step of outputting information related to the accepted measurement results and/or the cut-off value (408).

12. A computer program product comprising program code portions for performing the method according to any one of the preceding claims when the computer program product is executed on one or more computing devices.

13. An evaluation device (100, 500) adapted for evaluating measurement results for a multiplicity of biochemical markers related to a presence or absence of colorectal cancer in an individual (112, 512), comprising:

- a component (202) adapted to accept measurement results indicative of a concentration of each of the following markers:

o Seprase,
o CEA,
o Ferritin,

o OPNT, and
o anti-p53;

- a component (204) adapted to determine, based on the accepted measurement results, a score (410) related to the presence or absence of colorectal cancer in the individual (112,512),
wherein the determination includes

o calculating zero, one or more single terms, a single term representing the measurement result for exactly one marker, and
o calculating one or more interaction terms, an interaction term representing a multiplication of the measurement results for at least two of the markers;
o wherein each of the accepted measurement results for the markers is included in at least one of a single term and one or more interaction terms; and

- a component (206) adapted to output the determined score (410) as a basis for use in assessing the presence or absence of colorectal cancer.

14. The evaluation device of claim 13,
wherein the evaluation device (500) is remotely connectable with an analytical apparatus (502) adapted to perform measurements of a multiplicity of biochemical markers.

15. An analytical apparatus (102) comprising assays (106) for Seprase, CEA, Ferritin, OPNT, and Anti-P53, and further comprising an evaluation device (100) according to claim 13 or 14.

Fig. 1

Fig. 2

302 — Operation of evaluation device

304 — Accept measurement results of
Seprase, CEA, Ferritin, OPNT, and Anti-p53

306 — Determine a score related to the presence or absence of CRC,
wherein the determination includes calculating at least one
interaction term comprising a multiplication of the measurement
results for at least two of the markers

308 — Outputting the determined score as a basis for use in an
assessment related to CRC

310 — Return

Fig. 3

EP 2 426 615 A1

404

406

Name

ID

412

410

414

0.73

108

0

1

416

426

insignificant   significant

428

420

0.6

408

418

Spec. 95%

Sens. 68%

422

402

424

Fig. 4

Fig. 5

Europäisches
Patentamt

European
Patent Office

Office européen
des brevets

**EUROPEAN SEARCH REPORT**

Application Number

EP 10 00 8234

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2009/074276 A2 (ROCHE DIAGNOSTICS GMBH [DE]; HOFFMANN LA ROCHE [CH]; KARL JOHANN [DE];) 18 June 2009 (2009-06-18) * abstract; claims 1-15; example 4; tables 3,4 * * page 6, paragraph 4 - page 9, paragraph 9 * * page 26, paragraph 5 - page 27, paragraph 2 * | 1-15 | INV. G06F19/24 G01N33/574 |
| X | US 2007/269804 A1 (LIEW CHOONG-CHIN [CA] ET AL) 22 November 2007 (2007-11-22) * abstract * * paragraph [0002] - paragraph [0007] * * paragraph [0109] * * paragraph [0266] * * paragraph [0280] * * page 320 * | 1-15 | |
| A | US 2003/143520 A1 (HOOD LEROY E [US] ET AL) 31 July 2003 (2003-07-31) * paragraph [0075] - paragraph [0080] * | 1-15 | TECHNICAL FIELDS SEARCHED (IPC) G06F G01N |
| A | WO 02/061595 A1 (GENALYTICS INC [US] GENALYTICS INC [US]; KEHDER MATTHIAS [US]; DILLON) 8 August 2002 (2002-08-08) * abstract * * page 1, paragraph 2 - page 3, paragraph 3 * * page 15, paragraph 3 - page 16, paragraph 5 * | 1-15 | |

The present search report has been drawn up for all claims

| Place of search | Date of completion of the search | Examiner |
|---|---|---|
| The Hague | 20 October 2010 | Türkeli, Yasemin |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 10 00 8234

This annex lists the patent family members relating to the patent documents cited in the above-mentioned European search report.
The members are as contained in the European Patent Office EDP file on
The European Patent Office is in no way liable for these particulars which are merely given for the purpose of information.

20-10-2010

| Patent document cited in search report | | Publication date | Patent family member(s) | | Publication date |
|---|---|---|---|---|---|
| WO 2009074276 | A2 | 18-06-2009 | CA<br>EP<br>US | 2701970 A1<br>2223116 A2<br>2010240068 A1 | 18-06-2009<br>01-09-2010<br>23-09-2010 |
| US 2007269804 | A1 | 22-11-2007 | NONE | | |
| US 2003143520 | A1 | 31-07-2003 | WO | 03065034 A1 | 07-08-2003 |
| WO 02061595 | A1 | 08-08-2002 | CA<br>EP<br>JP<br>US | 2436352 A1<br>1352331 A1<br>2004530967 T<br>2003004903 A1 | 08-08-2002<br>15-10-2003<br>07-10-2004<br>02-01-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009074276 A2
- US 6414219 B
- US 5695761 A

**Non-patent literature cited in the description**

- **LEE et al.** *Blood,* 2006, vol. 107, 1397-1404
- **PIÑEIRO-SÁNCHEZ.** *J. Biol. Chem.,* 1997, vol. 272, 7595-7601
- **HENRY et al.** *Clin. Cancer Res.,* 2007, vol. 13, 1736-1741
- **GHERSI, G. et al.** *J. Biol. Chem.,* 2002, vol. 277, 29231-29241
- **GHERSI, G. et al.** *Adv. Exp. Med. Biol.,* 2003, vol. 524, 87-94
- **GHERSI, G. et al.** *Cancer Res.,* 2006, vol. 66, 4652-4661
- **GOLD, P. ; FREEDMAN, S.O.** *J. Exp Med,* 1965, vol. 121, 439-462
- Tumormarker und ihr sinnvoller Einsatz. Juergen Hartmann Verlag GmbH, 1993
- **WICK, M. et al.** Ferritin in Iron Metabolism - Diagnosis of Anemieas. Springer-Verlag, 1995
- Heterogeneity of ferritin II: Immunological aspects. **AROSIO, P. et al.** Ferritins and isoferritins as biochemical markers. Elsevier, 1984, 33-47
- **KALTWASSER, J.P. et al.** Serumferritin: Methodische und Klinische Aspekte. Springer Verlag, 1980
- **MORIKAWA, K. et al.** *Leuk. Lymphoma,* 1995, vol. 18 (5-6), 429-433
- **BORCH-IOHNSON, B.** *Analyst,* 1995, vol. 120 (3), 891-903
- **COOK, J. et al.** *Adv. Exp. Med. Biol.,* 1994, vol. 356, 219-228
- **NIITSU, Y. et al.** *Rinsho Ketsueki,* 1980, vol. 21, 1135-1143
- **KISHIDA, T. et al.** *J. Gastroenterol.,* 1994, vol. 29, 19-23
- **FENG, L. et al.** *J. Gastroenterol.,* 1999, vol. 34, 195-199
- **DENHARDT, D.T. ; GUO, X.** *FASEB J.,* 1993, vol. 7, 1475-1482
- **OLDBERG, A. et al.** *PNAS,* 1986, vol. 83, 8819-8823
- **OLDBERG, A. et al.** *J. Biol. Chem.,* 1988, vol. 263, 19433-19436
- **GIACHELLI, C.M. et al.** *Trends Cardiovasc. Med.,* 1995, vol. 5, 88-95
- **KIEFER M.C. et al.** *Nucl. Acids Res.,* 1989, vol. 17, 3306
- **WAI, P.Y. ; KUO, P.C.** *J. Surg. Res.,* 2004, vol. 121, 228-241
- **IRBY, R.B. et al.** *Clin. Exp. Metastasis,* 2004, vol. 21, 515-523
- **CRAWFORD, L. et al.** *Int. J. Cancer,* 1982, vol. 30, 403-408
- **BENCHIMOL, S. et al.** *EMBO J.,* 1982, vol. 1, 1055-1062
- **LEVINE, A.** *Cell,* 1997, vol. 88, 323-331
- **GREENBLATT, M. et al.** *Cancer Res.,* 1994, vol. 54, 4855-4878
- **SOUSSI, T.** *Cancer Res.,* 2000, vol. 60, 1777-1788
- **LUBIN.** *Cancer Res.,* 1993, vol. 53, 5872-5876
- **BODENMUELLER, H. et al.** *Int. J. Biol. Markers,* 1994, vol. 9, 75-81
- **STURGEON, C.** *Clinical Chemistry,* 2002, vol. 48, 1151-1159
- **VAN DER GAAST, A. et al.** *Br. J. Cancer,* 1994, vol. 69, 525-528